(19) **Europäisches Patentamt**
**European Patent Office**
**Office européen des brevets**

(11) **EP 4 436 476 B1**

(12) **EUROPEAN PATENT SPECIFICATION**

(45) Date of publication and mention
of the grant of the patent:
**03.09.2025   Bulletin 2025/36**

(21) Application number: **22821389.8**

(22) Date of filing: **21.11.2022**

(51) International Patent Classification (IPC):
*A61B 5/145* (2006.01)          *A61B 5/00* (2006.01)
*A61J 11/00* (2006.01)

(52) Cooperative Patent Classification (CPC):
**A61B 5/14507; A61B 5/4288;** A61J 11/0005

(86) International application number:
**PCT/EP2022/082584**

(87) International publication number:
**WO 2023/094312 (01.06.2023 Gazette 2023/22)**

(54) **A BREASTFEEDING ANALYSIS DEVICE AND SYSTEM**

VORRICHTUNG UND SYSTEM ZUR ANALYSE DES STILLENS

DISPOSITIF ET SYSTÈME D'ANALYSE DE L'ALLAITEMENT

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority:  **24.11.2021   EP 21210090**

(43) Date of publication of application:
**02.10.2024   Bulletin 2024/40**

(73) Proprietor: **ENT Research Clinic Horsens
8700 Horsens (DK)**

(72) Inventors:
• **CHIRIAEV, Serguei
6430 Nordborg (DK)**
• **LILDHOLDT, Torben
8700 Horsens (DK)**

(74) Representative: **Plougmann Vingtoft a/s
Strandvejen 70
2900 Hellerup (DK)**

(56) References cited:
WO-A1-2010/046812     US-A1- 2015 196 247
US-A1- 2015 297 464     US-A1- 2016 058 361
US-A1- 2018 116 913

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

FIELD OF THE INVENTION

The present invention relates to a breastfeeding device, decision support system and method for the analysis of breastfeeding and breastfeeding issues relating to infants.

BACKGROUND OF THE INVENTION

Breastfeeding during the early stage of life has been considered the golden standard for centuries. It is associated with protection against child infections, prevention of malocclusion, improved intelligence and probable reductions in overweight and diabetes. For nursing mothers breastfeeding gives protection against breast cancer and improves birth spacing and may protect against ovarian cancer and type 2 diabetes. Leading health authorities all recommend exclusive breastfeeding for at least six months' postpartum. Based on this, WHO and UNICEF have over many years published extensive guidelines for promoting breastfeeding.

Despite WHO's clear recommendations, the majority of mothers change to bottle feeding before 6 months. Universal exclusive breastfeeding for 6 months might prevent 823,000 annual deaths in children younger than 5 years and 20,000 annual deaths from breast cancer for adult women.

While most mothers initiate breastfeeding, many discontinue due to difficulties rather than maternal choice. One of the most common difficulties is nipple pain. A greater understanding of nipple pain and its treatment is needed to improve the breastfeeding period and exclusivity rates. Further research is warranted to evaluate the effectiveness of diverse interventions in order to reduce nipple pain.

Research suggests that nipple pain results from a mechanical overload of the nipple structures especially the skin and underlying connective tissue. To the applicant's knowledge, no device for measuring and analysing the forces that cause mechanical overload of the nipple is available. This might be accomplished by measuring the variations in pressure and shear forces generated mainly by the complex tongue movements that are required to drive the stream of milk from the nipple. A known, current method of analysing pressure and tongue movement of an infant is ultrasound imaging providing qualitative data. Other methods include measurements of the intraoral pressure, and measurements of the tongue displacements with strain gauges embedded into artificial nipples. These methods, however, do not provide sufficient quantitative data for biomechanical and statistical analysis.

One of the congenital anomalies, which disrupt breastfeeding, is ankyloglossia, often referred to as tongue-tie. Tongue-tie occurs due to tightness of the normal soft frenulum underneath the tongue. Normal movement of the tongue is impaired and breastfeeding poses problems: The baby gets insufficient milk, and the mother suffers from sore nipples. In clinically relevant cases, surgical treatment is instantly efficacious. Under local anesthesia, the tight frenulum is cut thereby relieving the movements of the tongue. Instantly, normal breastfeeding is possible with a relief for mother and baby.

However, the decision of treatment of tongue-tie depends on qualitative clinical observations by the surgeon, consequently an individual estimate. There exists no quantitative measurement to distinguish in advance, whom the surgery will help. Thus, there is much discrepancy between the existing qualitative diagnostic criteria and the results of surgical treatment. This is highlighted by an unexplained increase of the rate of surgical procedures for tongue-tie in USA and in Denmark. This underlines that the diagnosis and treatment of tongue-tie as well calls for clinical research.

US2015/0196247 A1 discloses a system, device and method for monitoring infant oral motor kinetics. The publication does not realize, that resilience of the nipple is an important adjustment parameter, when performing measurements.

WO2010/046812 A1 discloses a teat with a flexible wall, the wall having a pocket to receive a substance so as to alter the flexibility of the wall in a region of the pocket. The publication does not realize, that such pocket may be utilized to measure force.

Hence, a device for quantitative biomechanical analysis of the mouth of an infant's breastfeeding capabilities/technique would be advantageous, and in particular a more efficient and/or reliable method of assessing issues related to breastfeeding would be advantageous.

OBJECT OF THE INVENTION

In particular, it may be seen as an object of the present invention to provide a breastfeeding analysis device, method of analysis and a decision support system, which solve the above-mentioned problems of the prior art with breastfeeding and current clinical practice related to said problems.

SUMMARY OF THE INVENTION

Thus, the above-described object and several other objects are intended to be obtained in a first aspect of the invention by providing a breastfeeding device for an infant, said device comprising

- an artificial nipple comprising

  - a proximal end,
  - a distal end,
  - a cavity, said cavity being positioned within a wall portion of the artificial nipple, preferably at the distal end,
  - a first fluidic channel connected to the cavity,

- a pressure sensor fluidically connected to the first fluidic channel,
- a nipple resilience system configured to control resilience of the artificial nipple by controlling the resilience of the cavity, through the first fluidic channel, and

wherein the resilience of the artificial nipple is adjustable by the nipple resilience system and wherein the pressure sensor measures suckling pressure.

In another version of the first aspect, the invention provides a breastfeeding device for an infant, said device comprising

- an artificial nipple comprising

  - a proximal end,
  - a distal end,
  - a cavity, said cavity being positioned within a wall portion of the artificial nipple, preferably at the distal end,
  - a first fluidic channel connected to the cavity,

- a pressure sensor fluidically connected to the first fluidic channel,
- a nipple resilience system configured to control resilience of the artificial nipple by controlling the resilience of the cavity, through the first fluidic channel,
- data transmission means configured to transmit a signal from at least the pressure sensor, and

wherein the resilience of the artificial nipple is adjustable by the nipple resilience system and wherein the pressure sensor measures suckling pressure.

The invention is particularly, but not exclusively, advantageous for obtaining pressure data relating to an infant suckling or nursing on the artificial nipple. The invention may be advantageous for providing a physician, surgeon or other relevant health-care person with quantitative data sufficient to determine whether said infant may have a short tongue-tie, generates a weak pressure during nursing/suckling or displays other issues, which may hinder sufficient nutrient supply from the mother to the infant. Some infants may show dysfunctions in the force generation, rhythm or coordination of facial muscles. Furthermore, the invention provides a novel system wherein the cavity confined within the artificial nipple is configured to control the resilience of said artificial nipple while also being used to measure external forces applied to the cavity, through the interconnection of said cavity and the artificial nipple where the forces are applied from an external source, i.e. an infant or small child suckling on said artificial nipple.

In the context of the present invention, pressure is to be understood as positive and negative pressure provided by tongue and mouth and/or jaw movement by the infant during suckling/nursing. It is to be understood that pressure may be measured as absolute or relative pressure, and wherein relative pressure is pressure relative/differential to atmospheric pressure.

In the context of the present invention, suckling is to be understood as an infant's act of sucking, motioning and applying pressure to a nipple or artificial nipple, to stimulate the provision of milk from said nipple. It is further to be understood that the pressure applied by the infant can be both positive and negative in suckling cycles.

In the context of the present invention, an artificial nipple is to be understood as a synthetic nipple, i.e. a synthetic nipple shaped device, such as a nipple for a milk/formulae bottle.

In a preferred embodiment of the invention, the breastfeeding device further comprises data transmission means configured to transmit a signal from at least the pressure sensor. In another embodiment, the data transmission means are configured to transmit and/or receive a signal to/from at least one of the pressure sensor and the nipple resilience system.

In a preferred embodiment of the invention, the artificial nipple is made from a non-hazardous polymer or elastomer, such as silicone or natural rubber.

In another preferred embodiment, the artificial nipple is made from a monolithic element of polymer, such as silicone, the cavity being positioned within the silicone and wherein forces applied to the artificial nipple will translate to the at least one pressure sensor through the cavity connected to said pressure sensor. It is to be understood that the wall defining the cavity is an internal portion of the artificial nipple.

In yet another embodiment, the artificial nipple is made from a non-hazardous elastomer, such as a polyisoprene, preferably such as a natural rubber compound or latex.

In an advantageous embodiment, the artificial nipple is made from a non-gas-permeable material. In other embodiments, the cavity/cavities of the artificial nipple is coated with a non-gas-permeable or non-liquid-permeable material.

In some embodiments of the invention, the artificial nipple is moulded as a solid mass and wherein the cavity and fluidic channel is substantially the only exemption from said solid mass.

In the context of the present invention, cavity is to be understood as a hollow space enclosed by a wall section of the artificial nipple.

In a preferred embodiment of the invention, the cavity is non-permeable to fluids and suitable for containing gas and/or liquid within the cavity, thus, the gas and/or liquid within the cavity is supplied/removed through the fluidic channel.

In another preferred embodiment, the cavity comprises a balloon inserted within a hollow space or cavity within the artificial nipple. This embodiment may be particularly advantageous for providing a non-permeable barrier between a fluid within the balloon and the artificial nipple.

In yet another embodiment, a wall portion of the cavity, i.e. an internal wall portion of the artificial nipple may be coated with a non-permeable layer to prevent any fluid from seeping into the artificial nipple from within the cavity.

In the context of the present invention, a liquid is to be understood as a low to non-compressible substance, such as a liquid with the compressibility of saline or silicone oil. Preferably, the liquid is a non-hazardous, non-compressible liquid.

In one embodiment of the invention, the fluidic channel is defined by machining or moulding channels within the polymer of the artificial nipple.

In other embodiments, a fluidic line is inserted into the artificial nipple and connected to the cavity to provide a sealed connection between said fluidic line and cavity. The fluidic line may be made from any suitable material, such as a polymer, e.g. silicone, polyethylene, polytetrafluoroethylene or polyurethane.

In the context of the present invention, a pressure sensor is to be understood as a sensor or device, which converts pressure into a signal, such as a digital or analogue signal, i.e. a signal representing positive and/or negative pressure as a response to an external force or pressure. The pressure sensor may be a pressure transducer such as a piezoresistive transducer, a pressure gauge, a strain gauge pressure sensor, a potentiometric pressure sensor, an inductive pressure sensor, a capacitive pressure sensor, a piezoelectric pressure sensor, a variable reluctance pressure sensor or an optical pressure sensor.

In a preferred embodiment of the invention, the pressure sensor is a pressure transducer.

In the context of the present invention, resilience is to be understood as flexibility, elasticity or conformance, i.e. the resilience is the ability of an elastic material to absorb mechanical energy/force as a deformation, without breaking and to return from a deformed shape back to its original shape, when the mechanical energy/force is removed. The nipple resilience system within the present invention is particularly advantageous, as is transfers resilience from said system to the cavity within the artificial nipple, thus enabling the resilience of the artificial nipple to be adjustable by adjusting the resilience of the cavity within said artificial nipple.

In the context of the present invention, energy means is to be understood as a battery, such as a rechargeable battery, a non-replaceable battery or a replaceable battery. In some embodiments, energy means may be understood as a circuit configured to power the breastfeeding device using electromagnetic induction received from an auxiliary inductive device.

In yet other embodiments, the energy means is to be understood as a wired power connection, i.e. a power cord connected to the breastfeeding device, configured to supply power to the device.

In an advantageous embodiment of the invention, the nipple resilience system further comprises a resilience element, such as a bellows device, the resilience element being fluidically connected to the cavity in the artificial nipple, through the first fluidic channel, said resilience element having an adjustable resilience controlled by the resilience system and wherein adjusting the resilience of the resilience element translates to an adjusted resilience of the cavity and artificial nipple. This embodiment is particularly advantageous for providing a breastfeeding device in which, only one fluidic channel is necessary, to facilitate adjustable resilience of the cavity within the artificial nipple, and wherein the resilience of said cavity is adjusted by adjusting the resilience of the bellows device.

In the context of the present invention, bellows is to be understood as a device, which has adjustable deformation properties and configured so that changes in its ability to deform, under applied pressure/forces, results in changes in flexibility/softness/compliance of the cavity, and thus, of the artificial nipple. It is to be understood that the nipple resilience system and resilience element/bellows is connected to the cavity through the fluidic channel, and thus, any pressure applied to the artificial nipple will translate through said fluidic channel to the nipple resilience system and resilience element/-bellows.

In some embodiments, the bellows is a device comprising an elastic container filled with a fluid, and of elastic spring element(s), which is/are mechanically connected to the outer surface of the container and have an adjustable spring constant. The spring element(s) is/are configured to constrain the deformation of the container under the applied fluid pressure so that a decrease in the spring constant results in an increase of compliance of said container. The compliance of the container may be defined as a change in its volume per unit of pressure change. When the container is fluidically connected to the cavity, an increase in the container compliance results in an increase of the resilience of the cavity, and thus of the artificial nipple. The compliance may further be adjusted by removing or adding springs. The spring element may be any suitable type, such as a coil, cantilever, pneumatic or other.

In other embodiments, the bellows device comprises a flexible container within a non-flexible container, and wherein an actuator or motor is configured to change the volume of the non-flexible container, thus changing the rigidity of the flexible container within the non-flexible container by restricting/enabling expansion of the flexible container.

In another advantageous embodiment of the invention, the breastfeeding device further comprises a second fluidic channel fluidically connecting the cavity, the pressure sensor and the nipple resilience system, wherein the nipple resilience system is configured to adjust the resilience of the artificial nipple by adjusting a ratio of liquid to gas within the cavity of the artificial nipple, through the first and second fluidic channel. This embodiment is particularly advantageous for providing an easily adjustable nipple resilience system such as a valve system wherein one or more valves are used to control the ratio of liquid to gas within the cavity of the artificial nipple. It is to be understood that when providing a high ratio of non-compressible liquid to gas within the cavity, the flexibility of the artificial nipple decreases, thus providing a more rigid nipple. It is further to be understood that when increasing the ratio of gas to liquid within the cavity, the softness or compliance of the artificial nipple increases.

In yet another preferred embodiment of the invention, the nipple resilience system is a closed system, wherein no fluids can exit the system during operation and/or calibration and/or during storage, when not being operated. This embodiment is particularly advantageous for providing a more user-friendly system, which does not need to be refilled with neither liquid nor gas.

In other embodiments of the invention, the nipple resilience system further comprises a sealable opening for the provision of fluids, enabling the nipple resilience system to be refilled with fluids, such as a liquid and/or gas.

It is to be understood that the nipple resilience system, i.e. the adjustable resilience of the artificial nipple is particularly advantageous for providing a breastfeeding device, which is more accommodating to specific parameters of an individual infant, such as individual tongue strength, or suction/negative pressure generation of the individual infant. Thus, by adjusting the resilience of the artificial nipple to the specific physical strength or mouth/jaw features of the infant, the breastfeeding device provides data that are more accurate for the physician to diagnose said infant, such as for diagnosing tongue-tie or other issues relating to nursing.

In a preferred embodiment of the invention, the breastfeeding device further comprises processing means connected to the pressure sensor, nipple resilience system, and the data transmission means, said processing means being configured to calibrate the pressure sensor when/if the nipple resilience system adjusts the resilience of the artificial nipple and/or process instructions transmitted from an auxiliary device, such as instructions relating to the resilience of the artificial nipple. This embodiment is particularly advantageous for providing a breastfeeding device, which can be adjusted and calibrated through e.g. an associated electronic device, such as a smart-phone, tablet or computer using data transmission, such as wireless data transmission. Furthermore, the processing means may be configured to process signals measured from the pressure sensor.

It is to be understood that some embodiments of the pressure sensor may need calibration when the nipple resilience system has adjusted the resilience of the cavity.

It is further to be understood that the processing means may be selected from any type of suitable type of CPU, processor, processing device or computer.

In other preferred embodiments of the invention, the processing means and/or the breastfeeding device further comprises data storage means and wherein the breastfeeding device is configured to store measured pressure data and/or instructions, such as a range of test-parameters or set of instructions to a healthcare person, on said data storage means.

It is to be understood that test-parameters may be comprised of a range of temporal tests during which, the resilience of the artificial nipple is adjusted while an infant suckles on said artificial nipple.

It is further to be understood that the data storage means may be selected from e.g. a harddrive, a memory block, a memory circuit or other suitable memory device.

In other embodiments of the invention, the breastfeeding device is operated with e.g. buttons or a display mounted on a surface of the breastfeeding device.

In an advantageous embodiment of the invention, the one or more fluid channels further comprises fluid connectors at the proximal end of the artificial nipple configured to fluidically connect to respective fluidic connectors of the pressure sensor and/or nipple resilience system, enabling the artificial nipple to be detachable from the breastfeeding device. This embodiment is particularly advantageous for providing a two-part device with a detachable artificial nipple, such as if said artificial nipple malfunctions, another nipple size is needed to better suit the mouth size of the infant or the artificial nipple requires sterilization.

In some embodiments, the breastfeeding device is sealed and suitable for sterilization.

In yet another embodiment of the invention, the artificial nipple is disposable and/or recyclable.

In a preferred embodiment of the invention, the artificial nipple further comprises at least a second cavity positioned within the sidewall of said artificial nipple, said second cavity being fluidically connected to the nipple resilience system and the pressure sensor or to a secondpressure sensor, through one or more fluidic channels and/or fluidic connectors. This embodiment is particularly advantageous for providing pressure data from two or more positions on the artificial nipple.

It is to be understood that an infant may provide negative pressure with a jaw movement, thus creating negative pressure within the lumen of the oral orifice, while, at the same time, providing positive pressure (compressive) force, to at least a part of the artificial nipple, with the tongue. Thus, by providing at least two cavities within the artificial nipple, the device provides measurements of true/actual forces within the oral orifice of the infant, providing a better understanding any ailments or defects relating to nursing.

In some embodiments, when two or more cavities are present within the artificial nipple, the nipple resilience system is configured to adjust the resilience of each cavity separately, such as through individual fluidic channels connected to each cavity and wherein each of the cavities are not fluidically connected to each other.

In another preferred embodiment of the invention, a first and second cavity within the artificial nipple are positioned spaced apart, on a plane substantially parallel to a longitudinal axis of the artificial nipple, such as wherein the first cavity is positioned at the distal end of the artificial nipple and the second cavity is positioned between the proximal and distal end of the artificial nipple. This embodiment may provide the operator with detailed data regarding pressure waveforms generated by the infant's mouth over the length of a suckling cycle, when suckling on the artificial nipple thus providing better data to provide a physician with sufficient information to diagnose the infant.

In an advantageous embodiment of the invention, the artificial nipple further comprises a liquid canal, said liquid canal extending to an opening in the distal end of said artificial nipple, the liquid canal further being connected to an associated container at an end opposite to the distal end, said container containing a liquid, such as milk or formulae. This embodiment may be particularly advantageous for stimulating the infant to suckle on the breastfeeding device for a prolonged period of time to provide extensive data, such as for 1 to 10 minutes, such as for 20 to 30 minutes or such as for 30 to 60 minutes or longer.

In a more advantageous embodiment of the invention, the liquid canal further comprises

- a flow sensor configured to measure a flow of liquid within the liquid canal, and optionally
- pressure means to provide a pressurized flow of liquid from the container to the opening of the artificial nipple,

wherein the flow sensor and the optional pressure means are configured to receive/transmit data using the data transmission means and/or processing means.

This embodiment is particularly advantageous for providing a physician with additional data relating to the pressure provided by the infant to the artificial nipple. It is to be understood that when supplying a liquid to the mouth of the infant, the liquid may distort negative pressure data measured by the one or more pressure sensor, thus, by measuring the flow of liquid supplied to the infant's mouth, said data may be correlated to provide accurate pressure data. It is further to be understood that by providing pressurized liquid, such as milk or formulae to the infant, the infant may be incentivized to continue suckling for a prolonged time. Furthermore, the pressurized means may be used to train an infant to increase suckling pressure by training correct suckling patterns by increasing/decreasing milk flow during suckling cycles.

In the context of the present invention, correct suckling is to be understood as a suckling model obtained by analysing data from one or more infants with a healthy and/or sufficient suckling cycle.

In a preferred embodiment of the invention, the data transmission means are wireless data transmission means, and the breastfeeding device further comprises energy storage means configured to power one or more of, but not limited to, the pressure sensor, the nipple resilience system and processing means. This embodiment is particularly advantageous breastfeeding device, which is not restricted by wires and may be sealed for easier sterilization between uses.

In the context of the present invention, wireless data transmission means is to be understood as a device configured to enable the transfer of data between two or more points without the use of a wired electrical conductor as the medium for said data transfer. As an example, wireless data transmission means may be selected from standard radio wave communication, such as a Bluetooth device or a Wi-Fi device. Other examples may comprise ultrasonic data transfer, optical data transfer or electromagnetic induction data transfer.

Thus, the first aspect of the invention provides a breastfeeding device with one or more cavities within the artificial nipple and wherein the resilience of said artificial nipple is adjustable, by adjusting the resilience of said cavities and wherein the device may be configured to comprise several sizes of attachable/detachable artificial nipples for varying sizes of infants. Furthermore, the breastfeeding device provides pressure data through said cavities through the translation of hydraulic pressure/force from the one or more cavities to the one or more pressure sensors as any force applied to the artificial nipple is translated to the cavity positioned within the artificial nipple.

In a second aspect, the invention relates to a method of identifying suckling patterns from an infant, the method comprising

- providing a breastfeeding device according to the first aspect of the invention,
- positioning the artificial nipple within an infant's mouth,
- receiving measured data, such as pressure data, from the breastfeeding device to an auxiliary device, such as a computer or smart-phone device, and
- determining one or more patterns related to suckling from the infant providing pressure to the artificial nipple and the cavity during suckling.

In an advantageous embodiment of the invention, the method further comprises

- adjusting the resilience of the artificial nipple by adjusting the resilience of the one or more cavities in said artificial nipple based on the data received from one or more of

  - the pressure sensor(s), or
  - the flow sensor, and

- providing a new set of suckling patterns.

In another advantageous embodiment of the invention, the method further comprises

- adjusting the flow of liquid based on suckling data provided from the breastfeeding device by one or more of

  - reducing the flow of liquid,
  - increasing the flow of liquid, or
  - generating a pulsating flow, and

wherein the adjusted flow correlates to a suckling pattern of the infant.

This aspect of the invention is particularly, but not exclusively, advantageous in that the method according to the present invention may be implemented by continuously or intermittently adjusting one or more physical parameters relating to suckling/nursing, so as to accommodate the individual needs of infants. Thus, by adjusting the device to a specific infant, the device may provide a sufficient set of data to a physician, in order for said physician to reach a possible diagnosis. Optionally, the device may further enable a physician to train an infant to a more efficient method of suckling, should said infant's suckling method be insufficient to sustain and/or increase weight.

In some method embodiments, the breastfeeding device comprises an optimization step to reach a highest pressure differential through a measured suckling cycle, by adjusting the resilience of the artificial nipple and/or adjusting the liquid flow, until a maximum measured pressure differential through said suckling cycle is reached.

In a third aspect, the invention relates to a decision support system for identifying issues related to infant nursing, the system comprising

- a breastfeeding device according to the first aspect of the invention,
- carrying out the method according to the second aspect of the invention, and
- providing decision support to a healthcare person,

wherein the decision support system assists the healthcare person in adjusting the nursing/suckling based on data provided by the breastfeeding device, such as adjusting a position of the artificial nipple relative to an infant or adjusting a nursing position of said infant.

This embodiment is particularly advantageous for providing decision support to a physician, such as data required for said physician to make decisions relating to a diagnosis of an infant, e.g. tongue-tie or other illnesses/pathologies.

In a fourth aspect, the invention relates to a breastfeeding analysis kit for an infant, the kit comprising

- a breastfeeding device according to the first aspect of the invention,

- a computer program product enabling a computer system to identify suckling patterns based on data provided from the breastfeeding device,

wherein the computer program product provides data and/or decision support to a healthcare person, regarding infant nursing.

In a fifth aspect, the invention relates to the use of a breastfeeding device according to the first aspect of the invention.

In a sixth aspect, the invention relates to a medical diagnostic system, said system comprising the breastfeeding device according to the first aspect of the invention and wherein the system is configured to provide a diagnosis of an infant based on data provided from said breastfeeding device, when applying the method according to the second aspect of the invention.

The first, second, third, fourth, fifth and sixth aspect of the present invention may each be combined with any of the other aspects. These and other aspects of the invention will be apparent from and elucidated with reference to the embodiments described hereinafter.

BRIEF DESCRIPTION OF THE FIGURES

The device, system and method according to the invention will now be described in more detail with regard to the accompanying figures. The figures show one way of implementing the present invention and is not to be construed as being limiting to other possible embodiments falling within the scope of the attached claim set.

FIG. 1 is an illustration of the breastfeeding device 1, a liquid supply system LSS and an auxiliary device AUX_D, according to an embodiment of the invention.

FIG. 2 is a schematic illustration of the breastfeeding device 1, according to an embodiment of the invention.

FIG. 3 is a schematic illustration of the breastfeeding device 1, according to an alternative embodiment of the invention.

FIG. 4 is a schematic illustration of the breastfeeding device 1, according to yet another alternative embodiment of the invention.

FIG. 5 is an illustration of a side view cross-section of an artificial nipple 10, according to an embodiment of the invention.

FIG. 6 is an illustration of a side view cross-section of an artificial nipple 10 and schematics of a second part 20, according to an embodiment of the breastfeeding device 1.

FIG. 7 is a graph showing pressure measured over time, when applied to a prototype of the breastfeeding device 1, with a breast pump, according to an embodiment of the invention.

FIG. 8 is a graph showing pressure measured over time, when applied to a prototype, by an infant suckling on a prototype of the breastfeeding device 1, according to an embodiment of the invention.

Figure 9 is a flow-chart of a method according to the invention.

Figure 10 is a schematic presentation of a measured suckling pressure signal over time.

## DETAILED DESCRIPTION OF AN EMBODIMENT

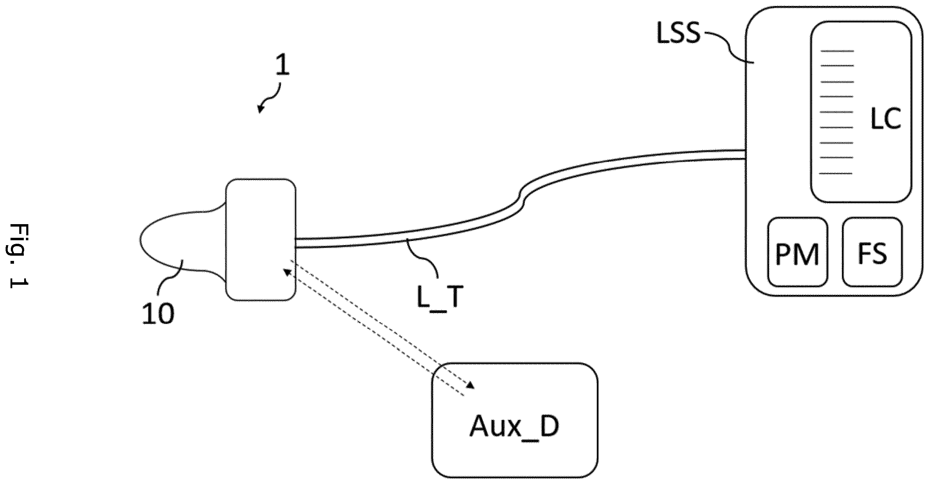

FIG. 1 shows an illustration of the breastfeeding device 1, a liquid supply system LSS and an auxiliary device Aux_D. The breastfeeding device 1 comprises an artificial nipple 10 and is fluidically connected, with a liquid tube L_T to the liquid supply system LSS. The liquid supply system LSS comprises a liquid container LC for containing a liquid, such as milk, water or formulae, pressure means PM and a flow sensor FS. In some embodiments, the flow sensor FS is integrated within the breastfeeding device 1. The liquid supply system LSS is configured to supply the liquid to the breastfeeding device 1 through the liquid tube L_T, and wherein the pressure means PM is configured to, optionally, pressurize the liquid provided to the breastfeeding device 1. The flow sensor FS is configured to measure the flow of liquid from the liquid container LC to the breastfeeding device 1. The breastfeeding device 1 and/or the liquid supply system LSS is configured to measure data relating to an infant (not shown) suckling on the artificial nipple 10, such as force applied to the artificial nipple 10 and flow of liquid provided from the liquid container LC, and provide the data (dotted arrows) to the auxiliary device Aux_D, such as a computer, smartphone or other suitable device.

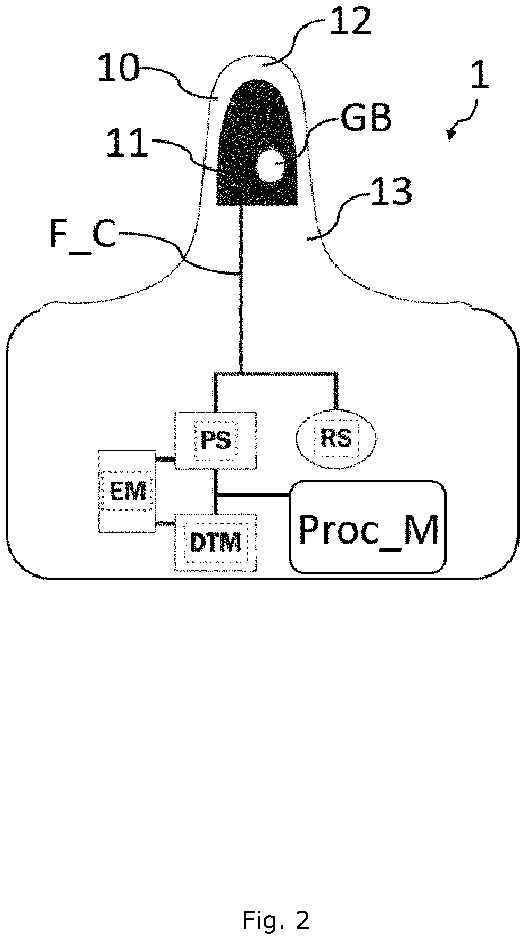

FIG. 2 shows a schematic illustration of the breastfeeding device 1. The breastfeeding device 1 comprises an artificial nipple 10, with a distal end 12 and a proximal end 13. A cavity 11 is positioned within the artificial nipple 10, said cavity 11 being fluidically connected to a pressure sensor PS and nipple resilience system RS, through a fluidic channel F_C. The white circle within the cavity 11 illustrates a gas bubble GB, and the black portion illustrates a non-compressible liquid. The nipple resilience system RS is controlled by processing means Proc_M. The processing means Proc_M is further connected to the pressure sensor PS to receive data from the pressure sensor PM and connected to data transmission means DTM to send/receive data to an auxiliary device (not shown). The breastfeeding device 1 further comprises energy means EM, configured to power the pressure sensor PS, nipple resilience system RS, data transmission means DTM and processing means Proc_M. The nipple resilience system RS is fluidically connected to the cavity 11 within the artificial nipple 10 through the fluidic channel F_C, to control the resilience of the artificial nipple 10, by adjusting a ratio of gas to liquid. To adjust the resilience of the artificial nipple 10, the nipple resilience system RS adjusts the ratio of gas to liquid, by e.g. purging gas and providing liquid to the cavity 11, to provide a more rigid cavity 11. The pressure sensor PS is configured to measure external force applied to the artificial nipple 10, as any force applied to the artificial nipple 10 translates to the cavity 11 which is fluidically connected to the pressure sensor PS.

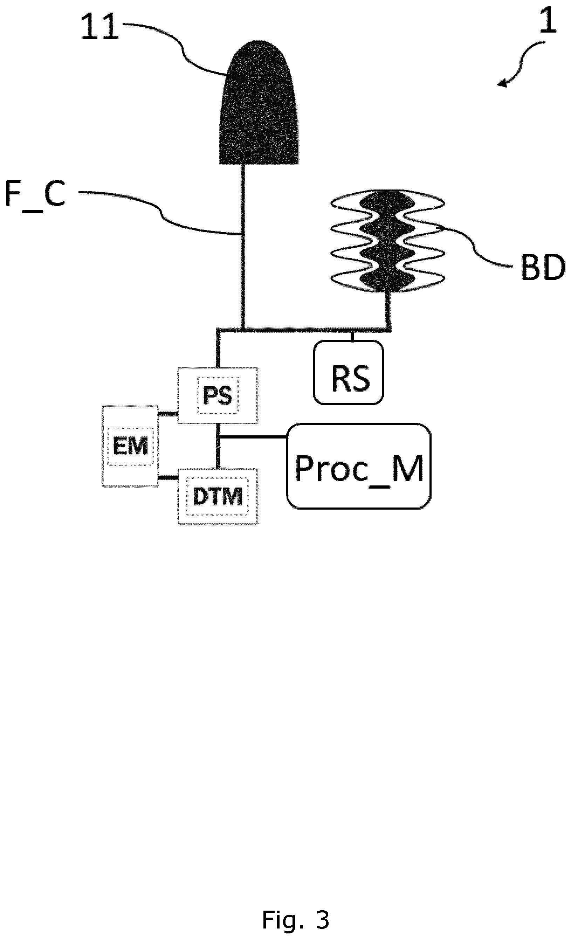

FIG. 3 shows a schematic illustration of the breastfeeding device 1. FIG. 3 shows a cavity 11 positioned within an artificial nipple (not shown), said cavity 11 being fluidically connected to a pressure sensor PS, a bellows device BD and nipple resilience system RS, through a fluidic channel F_C. The nipple resilience system RS is controlled by processing means Proc_M. The processing means Proc_M is further connected to the pressure sensor PS to receive data from the pressure sensor PM and connected to data transmission means DTM to send/receive data to an auxiliary device (not shown). The breastfeeding device 1 further comprises energy means EM, configured to power the pressure sensor PS, nipple resilience system RS, bellows device BD, data transmission means DTM and processing means Proc_M. The nipple resilience system RS is fluidically connected to the cavity 11 through the fluidic channel, to control the resilience of the cavity 11, by adjusting the resilience of the bellows device BD. The cavity 11, fluidic channel F_C and bellows device BD may be filled with a compressible gas. To adjust the resilience of the cavity 11, the nipple resilience system RS adjusts the resilience of the bellows device BD, by e.g. adjusting the volume of the bellows device BD, thus increasing or decreasing an internal pneumatic pressure within the cavity 11, to provide a more rigid or flexible cavity 11. The pressure sensor PS is configured to measure external force applied to the artificial nipple 10, as any force applied to the artificial nipple 10 translates to the cavity 11, which is fluidically connected to the pressure sensor PS. It is to be understood that the processing means is configured to calibrate the pressure sensor's PS output, based on the internal pressure within the cavity 11, when regulated by the nipple resilience system RS and bellows device BD.

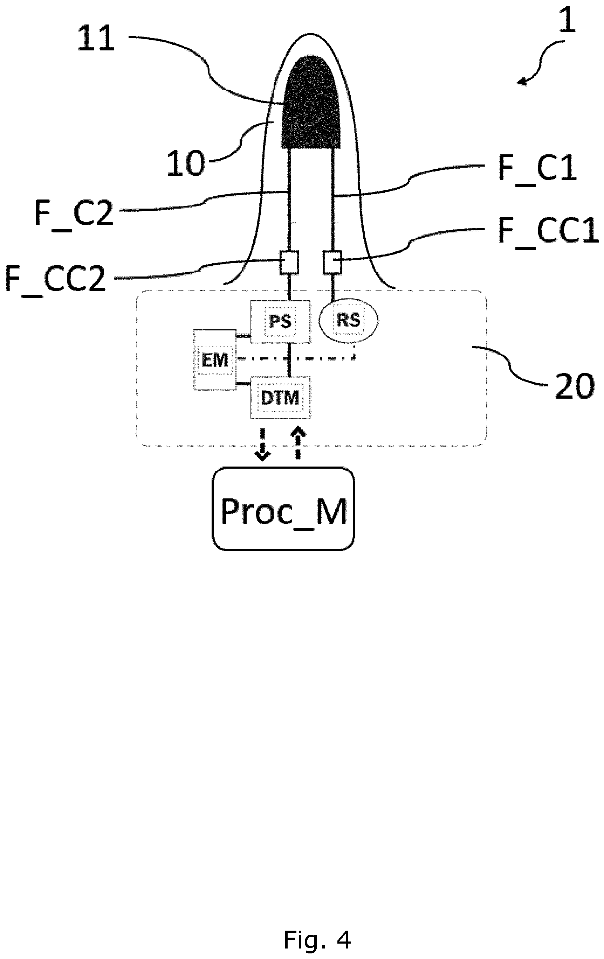

FIG. 4 shows a schematic illustration of the breastfeeding device 1 in a two-part setup. The breastfeeding device 1 comprises an artificial nipple 10 and a second part 20. In some embodiments, the artificial nipple 10 is disposable and the second part 20 is reusable. A cavity 11 is positioned within the artificial nipple 10, said cavity 11 being fluidically connected to a nipple resilience system RS through a first fluidic channel F_C1, the cavity 11 further being fluidically connected to a pressure sensor PS through a second fluidic channel F_C2. The first and second fluidic channel F_C1, F_C2 is configured with detachable connectors F_CC1, F_CC2, so as to enable the artificial nipple 10 and the second part 20 to be detachable from each other, such as for disposing of the artificial nipple 10 and attaching a second artificial nipple (not shown) to the second part 20. The nipple resilience system RS is controlled by processing means Proc_M, which may be integrated within the second part 20 or externally arranged to communicate with the nipple resilience system RS, energy means EM and pressure sensor PS through data transmission means DTM in the second part 20. The second part 20 comprises energy means EM, configured to power the pressure sensor PS, nipple resilience system RS, data transmission means DTM within the second 20 and optionally the processing means Proc_M, if positioned within the second part 20. The nipple resilience system RS is fluidically connected to the cavity 11 within the artificial nipple 10 through the fluidic channel F_C1, to control the resilience of the artificial nipple 10, such as by adjusting a ratio of gas to liquid. To adjust the resilience of the artificial nipple, the nipple resilience system RS may adjust the ratio of gas to liquid, by e.g. purging gas and providing liquid to the cavity 11, to provide a more rigid cavity 11. The pressure sensor PS is configured to measure external force applied to the artificial nipple 10, as any force applied to the artificial nipple 10 translates to the cavity 11, which is fluidically connected to the pressure sensor PS through the second fluidic channel F_C2. The connectors F_CC1, F_CC2 may be integrated within either the artificial nipple portion 100 or within the second part 20. It is to be understood that the connectors F_CC1, F_CC2 provide a fluidically sealed connection between the second part 20 and the artificial nipple 10, so as to enable secure replacement of either the second part 20 or the artificial nipple 10 of the breastfeeding device 1.

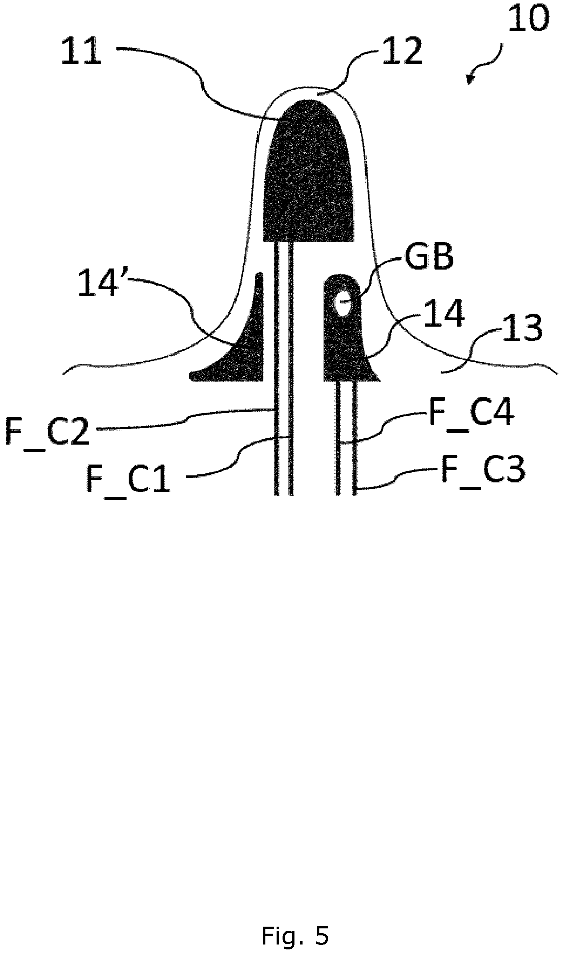

FIG. 5 is an illustration of a side view cross-section of an artificial nipple 10, according to an embodiment of the invention. The cross-sectional view shows two cavities 11, 14, 14' within the artificial nipple 10. The first cavity, 11 is located at a distal end 12 of the artificial nipple and the second cavity 14, 14' is located at a proximal end. It is to be understood that the second cavity 14, 14' is one cavity, shown in two parts, due to the cross-sectional view. Each of the cavities 11, 14, 14' comprises respective fluidic channels F_C1, F_C2, F_C3, F_C4, configured to be connected with a second part (not shown) of the breastfeeding device. The black portions within the cavities 11, 14, 14' represents a low to non-compressible liquid and the white circle within the second cavity 14 represents a gas bubble GB. Thus, within this embodiment of the artificial nipple, it is to be understood that the rigidity of the second cavity 14, 14' is lower than within the first cavity 11, due to said gas bubble, GB, which is compressible, relative to a non-compressible liquid. The artificial nipple may be manufactured from an elastic polymer, such as silicone, and wherein the cavities 11, 14, 14' resides within a solid silicone nipple encompassing said cavities 11, 14, 14'.

It is to be understood that a gas bubble GB may be present in either the first or second cavity 11, 14, 14', both or none of the cavities 11, 14, 14' depending on the needed resilience of the artificial nipple 10.

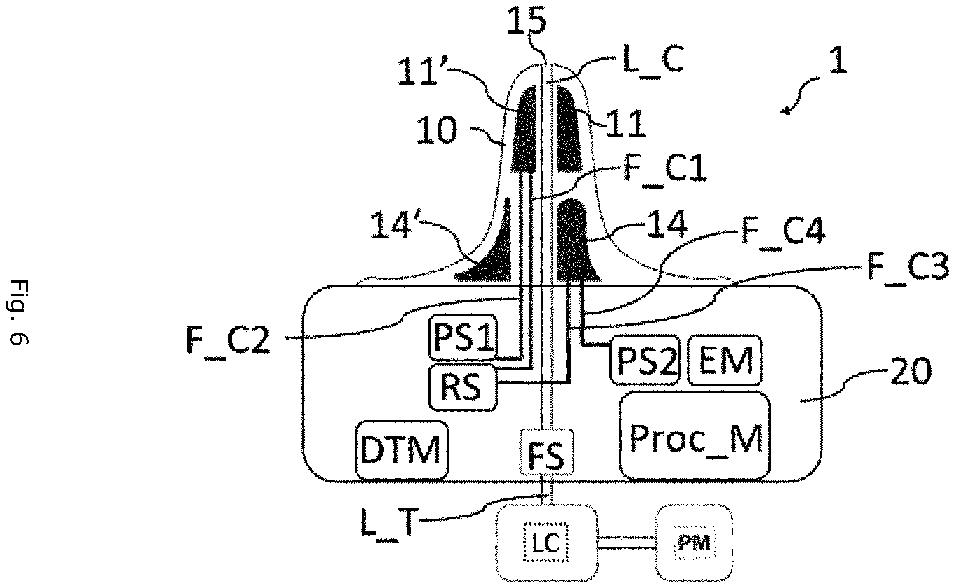

FIG. 6 is an illustration of a side view cross-section of an artificial nipple 10 and schematics of a second part 20, according to an embodiment of the breastfeeding device 1. The cross-sectional view of the artificial nipple shows two cavities 11, 14, 14' within the artificial nipple 10. It is to be understood that the first cavity 11, 11' and the second cavity 14, 14' each are shown in two parts, due to the cross-sectional view. Each of the cavities 11, 11', 14, 14' comprises respective fluidic channels F_C1, F_C2, F_C3, F_C4, configured to be connected with the second part 20 of the breastfeeding device 1. The first cavity 11, 11' is fluidically connected to the resilience system RS and a first pressure sensor PS1, through the first fluidic channel F_C1 and the second fluidic channel F_C2 respectively. The second cavity 14, 14' is fluidically connected to the resilience system RS and a second pressure sensor PS2, through the third fluidic channel F_C3 and the fourth fluidic channel F_C4 respectively. The artificial nipple 10 and the second part comprises a through-going liquid canal L_C configured to provide a liquid from an associated liquid container LC, through a liquid tube L_T inserted between the liquid container LC to the liquid canal L_C of the breastfeeding device 1, and provide said liquid to an opening 15 of the artificial nipple 10. The liquid container LC may be pressurized, by pressuring means PM, to provide pressurized liquid to the opening 15 of the artificial nipple. A flow sensor FS is positioned within the second part 20, configured to measure the flow of liquid from the liquid container LC to the opening 15 of the artificial nipple. It is to be understood that the flow sensor FS may be positioned external to the breastfeeding device 1, at any relevant position, such as at the liquid tube L_T or integrated in the liquid container LC. The second part 20 of the breastfeeding device 1 comprises data transmission means DTM to communicate with an auxiliary device, such as a smartphone. The second part 20 further comprises processing means configured to control the nipple resilience system RS and to communicate with the data transmission means DTM, the flow sensor FS, the first and second pressure sensors PS1, PS2 and the optional pressure means PM.

It is to be understood that the artificial nipple may comprise a single cavity or a plurality of cavities, such as two to eight cavities and any relevant number of pressure sensors to enable measuring of pressure provided to said plurality of cavities.

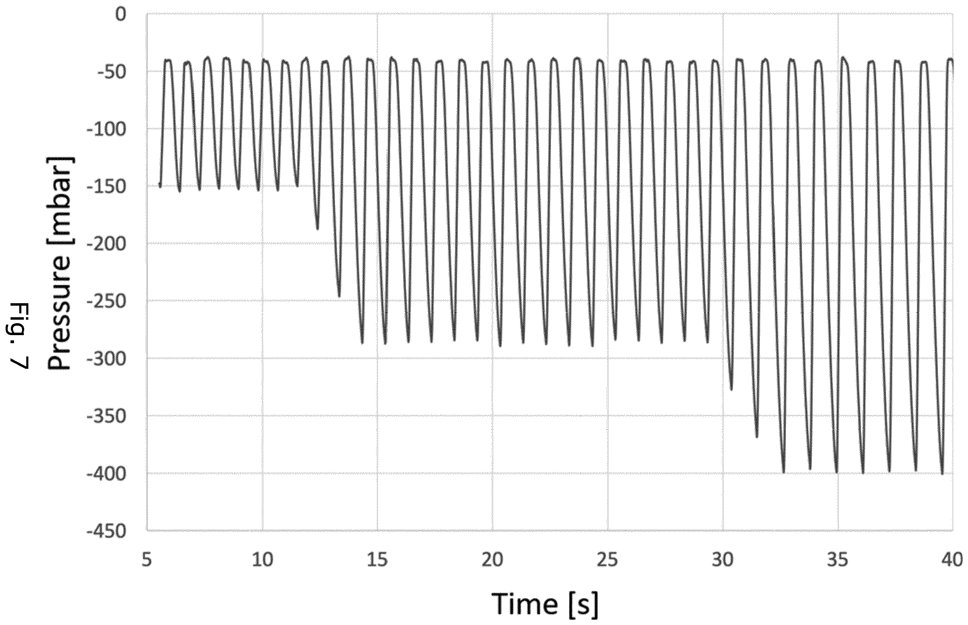

FIG. 7 is a graph showing pressure measured over time when applied to a prototype of the breastfeeding device 1, with a breast pump, according to an embodiment of the invention. The y-axis of the graph represents relative pressure in mbar, i.e. pressure relative to atmospheric pressure, provided by a breast pump attached to the artificial nipple of the breastfeeding device, and wherein the negative pressure value of the breast pump is incrementally increased to demonstrate correlating data measured from the breastfeeding device.

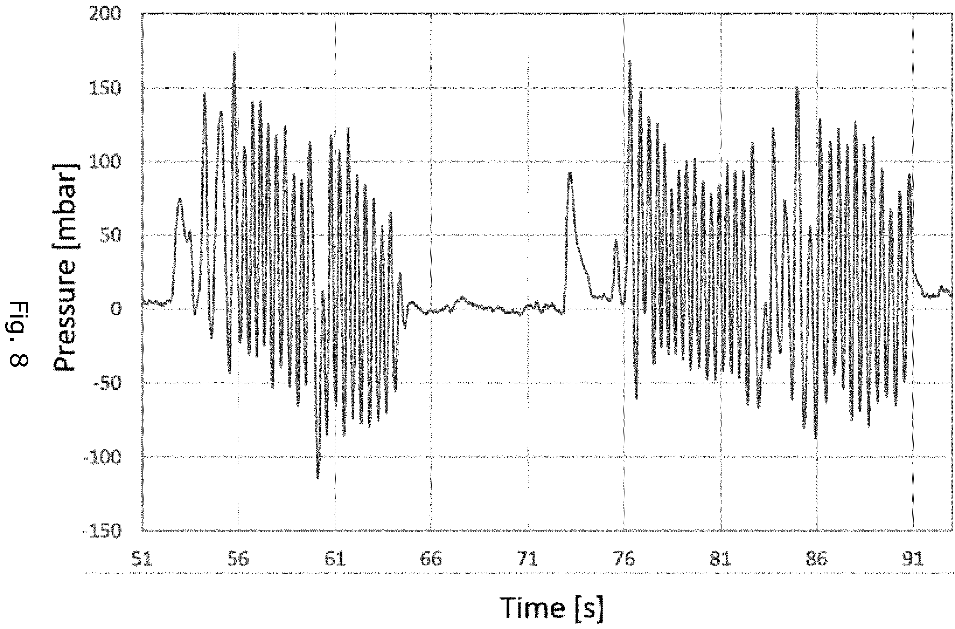

FIG. 8 is a graph showing pressure measured over time, when applied to a prototype, by an infant suckling on a prototype of the breastfeeding device 1, according to an embodiment of the invention. The y-axis of the graph represents relative pressure in mbar, provided by an infant suckling on artificial nipple of the breastfeeding device, and wherein the negative pressure force represents negative pressure generated within the oral orifice of the infant by jaw movements, and the positive pressure represents compression performed by the tongue and jaw on said artificial nipple. From the graph, it is visible that the infants suckles between approximately 52 second, represented on the x-axis, to approximately 65 second, pauses and then initiates suckling again at approximately 72 second on the x-axis.

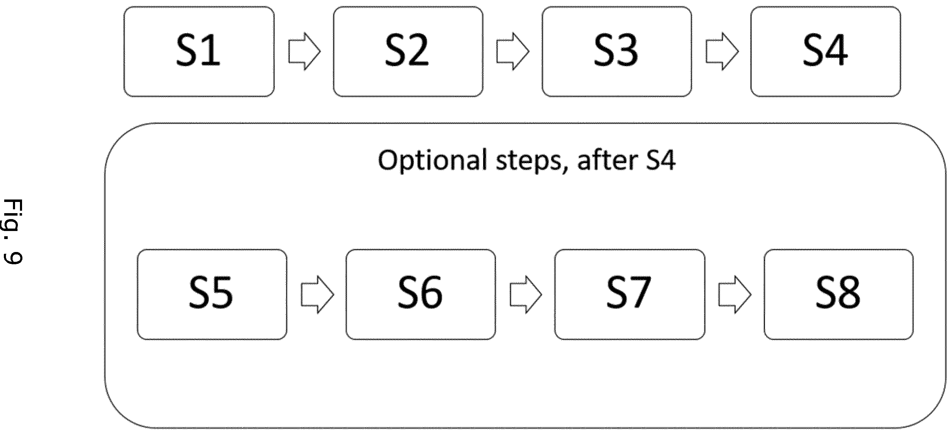

FIG. 9 shows a flowchart of a method of identifying suckling patterns from an infant, according to the invention, showing the following steps:

> **S1**-providing a breastfeeding device 1 according to the first aspect of the invention,
> **S2**-positioning the artificial nipple 10 within an infant's mouth,
> **S3**-receiving measured data, such as pressure data, from the breastfeeding device 1 to an auxiliary device Aux_D, such as a computer or smart-phone device,
> **S4**-determining one or more patterns related to suckling from the infant providing pressure to the artificial nipple 10 and the cavity 11, 14 during suckling, and optionally one or more of the steps:
> **S5**-adjusting the resilience of the artificial nipple 10 by adjusting the resilience of the one or more cavities 11, 14 in said artificial nipple 10 based on the data received from one or more of
>
> - the pressure sensor(s) PS1, PS2, or
> - the flow sensor FS,
>
> **S6**-providing a new set of suckling patterns,
> **S7**-adjusting the flow of liquid based on suckling data provided from the breastfeeding device 1 by one or more of
>
> - reducing the flow of liquid,
> - increasing the flow of liquid, or
> - generating a pulsating flow, and
>
> **S8**-providing yet a new set of suckling patterns.

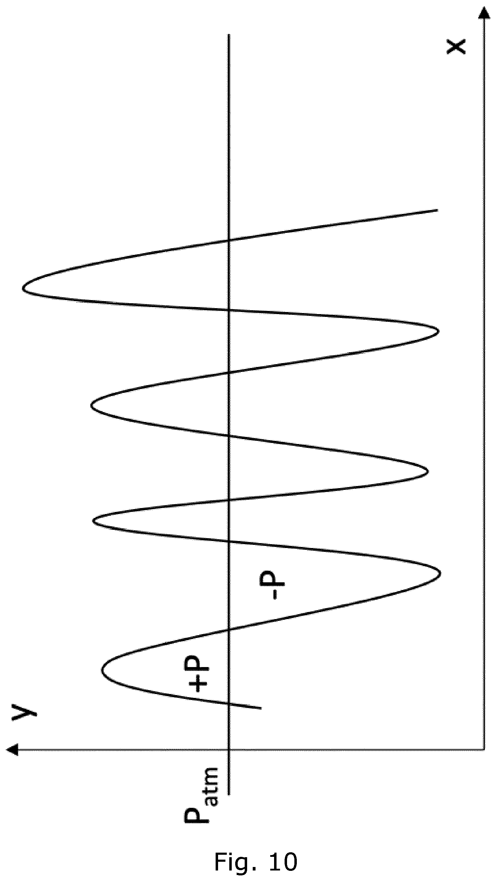

FIG. 10 is a schematic presentation of a measured suckling pressure signal over time. The figure shows atmospheric pressure Patm on the y-axis and time on the x-axis. In this figure, +P indicates a positive pressure and -P indicates a negative pressure, relative to the atmospheric pressure. Pressure peaks denoted in this figure as +P and -P constitute one pressure oscillation.

Signal analysis:

For the diagnostic purposes the known parameters, such as the amplitude and the frequency of the pressure oscillations shown in FIG. 8 are determined. In addition, a parameter is introduced, denoted by S, as the total suckling strength of an infant, given as:

$$S = \int |P_r| \, dt/N,$$

where $|P_r|$ is an absolute value of the pressure applied to one of the device cavities and measured as the pressure relative to the atmospheric pressure Patm. And N is the number of oscillations within the performed suckling measurement.

In other terms, the integral in the above expression is the total area under the suckling peaks +P, -P occurring in both positive and negative pressure regions and the area is proportional to the energy spent by the infant during a suckling measurement. Dividing by N gives an average value of the total area per one oscillation.

In order to quantify the contribution of the negative pressure into the suckling process the following parameters are introduced:

> a) Strength in the negative pressure region, defined as $S^{(-)} = \int |P_{(-)}| \, dt/N$, where
> $|P_{(-)}|$ corresponds to the absolute values of pressure smaller than the atmospheric pressure, and
>
> b) Ratio $S^{(-)}/S$, which gives the suckling strength in the negative pressure region relative to the total suckling strength.

In short, the invention relates to a breastfeeding device 1 for an infant, the device enabling analysis of true forces applied by the infants tongue and jaw movements during suckling, i.e. when suckling milk or formulae for the purpose of nourishment. The breastfeeding device 1 comprises an artificial nipple 10 with at least one cavity 11 within said artificial nipple 10, encompassed by the material of the artificial nipple 10. The cavity 11 is fluidically connected to a resilience system RS, enabling control of the resilience of the artificial nipple 10 by adjusting the resilience of the at least one cavity 11 through the principle of hydraulics. The cavity 11 is further fluidically connected to a pressure sensor PS enabling measurement of any force applied to the artificial nipple 10, and the measurements from the pressure sensor is provided to a user, such as a paediatrician, for decision support relating to diagnosis of the infant.

The individual elements of an embodiment of the invention may be physically, functionally and logically implemented in any suitable way such as in a single unit, in a plurality of units or as part of separate functional units. The invention may be implemented in a single unit, or be both physically and functionally distributed between different units and processors.

Although the present invention has been described in connection with the specified embodiments, it should not be construed as being in any way limited to the presented examples. The scope of the present invention is set out by the accompanying claim set. In the context of the claims, the terms "comprising" or "comprises" do not exclude other possible elements or steps. Also, the mentioning of references such as "a" or "an" etc. should not be construed as excluding a plurality. The use of reference signs in the claims with respect to elements indicated in the figures shall also not be construed as limiting the scope of the invention. Furthermore, individual features mentioned in different claims, may possibly be advantageously combined, and the mentioning of these features in different claims does not exclude that a combination of features is not possible and advantageous.

**Claims**

1. A breastfeeding device (1) for an infant, said device comprising

    - an artificial nipple (10) comprising

        - a proximal end (13),
        - a distal end (12),
        - a cavity (11), said cavity being positioned within a wall portion of the artificial nipple, preferably at the distal end,
        - a first fluidic channel (F_C) connected to the cavity,

    - a pressure sensor (PS) fluidically connected to the first fluidic channel,

    **characterized by**

        - a nipple resilience system (RS) configured to control resilience of the artificial nipple by controlling the resilience of the cavity, through the first fluidic channel,

    wherein the resilience of the artificial nipple is adjustable by the nipple resilience system and wherein the pressure sensor measure suckling pressure.

2. The breastfeeding device according to claim 1 further comprising data transmission means configured to transmit a signal from at least the pressure sensor.

3. The breastfeeding device according to claim 1 or 2, the nipple resilience system further comprising a resilience element, such as a bellows device (BD), the resilience element being fluidically connected to the cavity in the artificial nipple, through the first fluidic channel, said resilience element having an adjustable resilience controlled by the nipple resilience system and wherein adjusting the resilience of the resilience element translates to an adjusted resilience of the cavity and artificial nipple.

4. The breastfeeding device according to any of the preceding claims further comprising

    - a second fluidic channel (F_C2) fluidically connecting the cavity, the pressure sensor and the nipple resilience system, the nipple resilience system configured to adjust the resilience of the artificial nipple by adjusting a ratio of liquid to gas within the cavity of the artificial nipple, through the first and second fluidic channel.

5. The breastfeeding device according to any of the preceding claims further comprising processing means (Proc_M) connected to the pressure sensor and the nipple resilience system, said processing means being configured to calibrate the pressure sensor when/if the nipple resilience system adjust the resilience of the artificial nipple.

6. The breastfeeding device according to claim 5, said processing means further being connected to the data transmission means according to claim 2, the processing means being configured to process instructions transmitted and/or received from/to an auxiliary device (Aux_D), such as instructions relating to the resilience of the artificial nipple.

7. The breastfeeding device according to any of the preceding claims, the fluid channel(s) further comprising fluid connectors (F_CC1, F_CC2) at the proximal end of the artificial nipple configured to fluidically connect to respective fluidic connectors of the pressure sensor and/or nipple resilience system, enabling the artificial nipple to be detachable from the breastfeeding device.

8. The breastfeeding device according to any of the preceding claims, the artificial nipple further comprising at least a second cavity (14) positioned within the sidewall of said artificial nipple, said second cavity being fluidically connected to the nipple resilience system and the pressure sensor or to a secondary pressure sensor (PS2), through one or more fluidic channels and/or fluidic connectors.

9. The breastfeeding device according to claim 8, wherein a first and second cavity within the artificial nipple are positioned spaced apart, on a plane substantially parallel to a longitudinal axis of the artificial nipple, such as wherein the first cavity is positioned at the distal end of the artificial nipple and the second cavity is positioned between the proximal and distal end of the artificial nipple.

10. The breastfeeding device according to any of the preceding claims, the artificial nipple further comprising a liquid canal (L_C), said liquid canal extending to an opening in the distal end of said artificial nipple, the liquid canal further being connected to an associated container (LC) at an end opposite to the distal end, said container containing a liquid, such as milk or formulae.

11. The breastfeeding device according to claim 10, the liquid canal further comprising a flow sensor (FS) configured to measure a flow of liquid within the liquid canal or pressure means (PM) configured to provide a pressurized flow of liquid from the container to the opening of the artificial nipple.

12. The breastfeeding device according to claim 11, wherein the flow sensor or the pressure means are configured to receive/transmit data using the data transmission means according to claim 2 and/or the processing means according to claim 5.

13. The breastfeeding device according to claim 2 wherein the data transmission means is wireless data transmission means and the breastfeeding device further comprises energy storage means (EM) configured to power one or more of, but not limited to, the pressure sensor, the nipple resilience system and/or the processing means according to claim 5.

14. A method of identifying suckling patterns from an infant, the method comprising

- providing a breastfeeding device (1) according to any of claims 1 to 13,
- positioning the artificial nipple (10) within an infant's mouth,
- measuring suckling on the artificial nipple from the infant,
- determining one or more patterns related to suckling from the infant providing pressure to the artificial nipple and the cavity (11, 14) during suckling.

15. A breastfeeding analysis kit for an infant, the kit comprising

- a breastfeeding device according to any of claim 1 to 13,
- a computer program product enabling a computer system to identify suckling patterns based on data provided from the breastfeeding device, wherein the computer program product provides data and/or decision support to a healthcare person, regarding infant nursing.

**Patentansprüche**

1. Stillvorrichtung (1) für einen Säugling, wobei die Vorrichtung umfasst

- eine künstliche Brustwarze (10), umfassend

- ein proximales Ende (13),
- ein distales Ende (12),
- einen Hohlraum (11), wobei der Hohlraum innerhalb eines Wandabschnitts der künstlichen Brustwarze, vorzugsweise am distalen Ende, angeordnet ist,
- einen ersten Fluidkanal (F_C), der mit der Kavität verbunden ist,

- einen Drucksensor (PS), der mit dem ersten Fluidkanal fluidisch verbunden ist,

**gekennzeichnet durch**

- ein Brustwarzen- Elastizitätssystem (RS), das so konfiguriert ist, dass es eine Elastizität der künstlichen Brustwarze durch Steuerung der Elastizität des Hohlraums über den ersten Fluidkanal steuert,

wobei die Elastizität der künstlichen Brustwarze durch das Brustwarzen-Elastizitätssystem einstellbar ist und wobei der Drucksensor einen Saugdruck misst.

2. Stillvorrichtung nach Anspruch 1, weiter umfassend eine Datenübertragungseinrichtung, die dazu konfiguriert ist, ein Signal von zumindest dem Drucksensor zu übertragen.

3. Stillvorrichtung nach Anspruch 1 oder 2, wobei das Brustwarzen-Elastizitätssystem weiter ein Elastizitätselement, beispielsweise eine Balgvorrichtung (BD), umfasst, wobei das Elastizitätselement durch den ersten Fluidkanal fluidisch mit dem Hohlraum in der künstlichen Brustwarze verbunden ist, wobei das Elastizitätselement eine einstellbare Elastizität aufweist, die durch das Brustwarzen-Elastizitätssystem gesteuert wird, und wobei sich ein Einstellen der Elastizität des Elastizitätselements in eine eingestellte Elastizität des Hohlraums und der künstlichen Brustwarze übersetzt.

4. Stillvorrichtung nach einem der vorstehenden Ansprüche, weiter umfassend

- einen zweiten Fluidkanal (F_C2), der den Hohlraum, den Drucksensor und das Brustwarzen-Elastizitätssystem fluidisch verbindet, wobei das Brustwarzen-Elastizitätssystem so konfiguriert ist, dass es die Elastizität der künstlichen Brustwarze durch Einstellen eines Verhältnisses von Flüssigkeit zu Gas innerhalb des Hohlraums der künstlichen Brustwarze durch den ersten und zweiten Fluidkanal einstellt.

5. Stillvorrichtung nach einem der vorstehenden Ansprüche, weiter umfassend eine Verarbeitungseinrichtung (Proc_M), die mit dem Drucksensor und dem Brustwarzen-Elastizitätssystem verbunden ist, wobei die Verarbeitungseinrichtung dazu konfiguriert ist, den Drucksensor zu kalibrieren, wenn/falls das Brustwarzen-Elastizitätssystem die Elastizität der künstlichen Brustwarze einstellt.

6. Stillvorrichtung nach Anspruch 5, wobei die Verarbeitungseinrichtung weiter mit der Datenübertragungseinrichtung nach Anspruch 2 verbunden ist, die Verarbeitungseinrichtung dazu konfiguriert ist, von einer Zusatzvorrichtung (Aux_D) übertragene und/oder empfangene Anweisungen zu verarbeiten, wie etwa Anweisungen bezüglich der Elastizität der künstlichen Brustwarze.

7. Stillvorrichtung nach einem der vorstehenden Ansprüche, wobei der Fluidkanal/die Fluidkanäle weiter Fluidanschlüsse (F_CC1, F_CC2) am proximalen Ende der künstlichen Brustwarzen umfasst/umfassen, die so konfiguriert sind, dass sie eine Fluidverbindung mit den jeweiligen Fluidanschlüssen des Drucksensors und/oder des Brustwarzen-Elastizitätssystems herstellen, wodurch die künstliche Brustwarze von der Stillvorrichtung abgenommen werden kann.

8. Stillvorrichtung nach einem der vorstehenden Ansprüche, wobei die künstliche Brustwarze weiter mindestens einen zweiten Hohlraum (14) umfasst, der in der Seitenwand der künstlichen Brustwarze angeordnet ist, wobei der zweite Hohlraum durch einen oder mehrere Fluidkanäle und/oder Fluidanschlüsse fluidisch mit dem Brustwarzen-Elastizitätssystem und dem Drucksensor oder einem sekundären Drucksensor (PS2) verbunden ist.

9. Stillvorrichtung nach Anspruch 8, wobei ein erster und ein zweiter Hohlraum innerhalb der künstlichen Brustwarze mit Abstand voneinander angeordnet sind, auf einer Ebene, die im Wesentlichen parallel zu einer Längsachse der künstlichen Brustwarze verläuft, wobei beispielsweise der erste Hohlraum am distalen Ende der künstlichen Brustwarze angeordnet ist und der zweite Hohlraum zwischen dem proximalen und distalen Ende der künstlichen Brustwarze angeordnet ist.

10. Stillvorrichtung nach einem der vorstehenden Ansprüche, wobei die künstliche Brustwarze weiter einen Fluidkanal (L_C) umfasst, wobei sich der Fluidkanal bis zu einer Öffnung im distalen Ende der künstlichen Brustwarze erstreckt, wobei der Fluidkanal weiter an einem dem distalen Ende gegenüberliegenden Ende mit einem zugehörigen Behälter (LC) verbunden ist, wobei der Behälter eine Flüssigkeit wie etwa Milch oder Muttermilchersatz enthält.

11. Stillvorrichtung nach Anspruch 10, wobei der Fluidkanal weiter einen Durchflusssensor (FS) umfasst, der dazu konfiguriert ist, einen Flüssigkeitsfluss innerhalb des Fluidkanals zu messen, oder eine Druckeinrichtung (PM) umfasst, die dazu konfiguriert ist, einen unter Druck stehenden Flüssigkeitsfluss vom Behälter zur Öffnung der künstlichen Brustwarze bereitzustellen.

12. Stillvorrichtung nach Anspruch 11, wobei der Durchflusssensor oder die Druckeinrichtung dazu konfiguriert sind, Daten unter Verwendung der Datenübertragungseinrichtung nach Anspruch 2 und/oder der Verarbeitungseinrichtung nach Anspruch 5 zu empfangen/zu übertragen.

13. Stillvorrichtung nach Anspruch 2, wobei die Datenübertragungseinrichtung eine drahtlose Datenübertragungseinrichtung ist und die Stillvorrichtung weiter eine Energiespeichereinrichtung (EM) umfasst, die dazu konfiguriert ist, einen oder mehrere von dem Drucksensor, dem Brustwarzen-Elastizitätssystem und/oder der Verarbeitungseinrichtung nach Anspruch 5 mit Strom zu versorgen, jedoch nicht darauf beschränkt ist.

14. Verfahren zum Erkennen von Saugmustern von einem Säugling, wobei das Verfahren umfasst

   - Bereitstellen einer Stillvorrichtung (1) nach einem der Ansprüche 1 bis 13,
   - Positionieren der künstlichen Brustwarze (10) in einem Mund eines Säuglings,
   - Messung eines Saugens des Säuglings an der künstlichen Brustwarze,
   - Bestimmen eines oder mehrerer Muster im Zusammenhang mit einem Saugen des Säuglings, indem er während eins Saugens Druck auf die künstliche Brustwarze und den Hohlraum (11, 14) ausübt.

15. Stillanalyse-Kit für einen Säugling, wobei das Kit umfasst

   - eine Stillvorrichtung nach einem der Ansprüche 1 bis 13,
   - ein Computerprogrammprodukt, das es einem Computersystem ermöglicht, Saugmuster auf der Grundlage von Daten der Stillvorrichtung zu erkennen,

wobei das Computerprogrammprodukt einer im Gesundheitswesen tätigen Person Daten und/oder Entscheidungshilfen in Bezug auf eine Säuglingspflege bereitstellt.

**Revendications**

1. Dispositif d'allaitement (1) pour un nourrisson, comprenant

    - un mamelon artificiel (10) comprenant

        - une extrémité proximale (13),
        - une extrémité distale (12),
        - une cavité (11), ladite cavité étant positionnée à l'intérieur d'une partie de paroi du mamelon artificiel, de préférence à l'extrémité distale,
        - un premier canal fluidique (F_C) relié à la cavité,

    - un capteur de pression (PS) en communication fluidique avec le premier canal fluidique,

    **caractérisé par**

        - un système de résilience (RS) de mamelon, configuré pour régler la résilience du mamelon artificiel en réglant la résilience de la cavité, via le premier canal fluidique,

    dans lequel la résilience du mamelon artificielle est ajustable par le système de résilience de mamelon et dans lequel le capteur de pression mesure la pression de succion.

2. Dispositif d'allaitement selon la revendication 1, comprenant en outre un moyen de transmission de données configuré pour transmettre un signal provenant d'au moins le capteur de pression.

3. Dispositif d'allaitement selon la revendication 1 ou 2, le système de résilience de mamelon comprenant en outre un élément de résilience, tel qu'un dispositif à soufflet (BD), l'élément de résilience étant en communication fluidique avec la cavité dans le mamelon artificiel, via le premier canal fluidique, ledit élément de résilience ayant une résilience ajustable réglée par le système de résilience de mamelon et dans lequel l'ajustement de la résilience de l'élément de résilience se traduit par une résilience ajustée de la cavité et du mamelon artificiel.

4. Dispositif d'allaitement selon l'une quelconque des revendications précédentes, comprenant en outre

    - un second canal fluidique (F_C2) en communication fluidique avec la cavité, le capteur de pression et le système de résilience de mamelon, le système de résilience de mamelon étant configuré pour ajuster la résilience du mamelon artificiel en ajustant un rapport de liquide à gaz à l'intérieur de la cavité du mamelon artificiel, via le premier et le second canal fluidique.

5. Dispositif d'allaitement selon l'une quelconque des revendications précédentes, comprenant en outre un moyen de traitement (Proc_M) relié au capteur de pression et au système de résilience de mamelon, ledit moyen de traitement étant configuré pour calibrer le capteur de pression lorsque/si le système de résilience de mamelon ajuste la résilience du mamelon artificiel.

6. Dispositif d'allaitement selon la revendication 5, ledit moyen de traitement étant en outre relié au moyen de transmission de données selon la revendication 2, le moyen de traitement étant configuré pour traiter des instructions transmises à et/ou reçues par un dispositif auxiliaire (Aux_D), telles que des instructions relatives à la résilience du mamelon artificiel.

7. Dispositif d'allaitement selon l'une quelconque des revendications précédentes, le canal/les canaux fluidique(s) comprenant en outre des connecteurs fluidiques (F_CC1, F_CC2) à l'extrémité proximale du mamelon artificiel, configurés pour être en communication fluidique avec des connecteurs fluidiques respectifs du capteur de pression et/ou du système de résilience de mamelon, permettant au mamelon artificiel d'être détachable du dispositif d'allaitement.

8. Dispositif d'allaitement selon l'une quelconque des revendications précédentes, le mamelon artificiel comprenant en outre au moins une seconde cavité (14) positionnée à l'intérieur de la paroi latérale dudit mamelon artificiel, ladite seconde cavité étant en communication fluidique avec le système de résilience de mamelon et le capteur de pression ou avec un capteur de pression secondaire (PS2), via un ou plusieurs canaux fluidiques et/ou connecteurs fluidiques.

9. Dispositif d'allaitement selon la revendication 8, dans lequel une première et une seconde cavité à l'intérieur du mamelon artificiel sont positionnées à distance l'une de l'autre, sur un plan sensiblement parallèle à un axe longitudinal du mamelon artificiel, par exemple dans lequel la première cavité est positionnée à l'extrémité distale du mamelon artificiel et la seconde cavité est positionnée entre l'extrémité proximale et l'extrémité distale du mamelon artificiel.

10. Dispositif d'allaitement selon l'une quelconque des revendications précédentes, le mamelon artificiel comprenant en outre un canal à liquide (L_C), ledit canal à liquide s'étendant jusqu'à un orifice dans l'extrémité distale dudit mamelon artificiel, le canal à liquide étant en outre relié à un récipient associé (LC) à une extrémité opposée à l'extrémité distale, ledit récipient contenant un liquide, tel que du lait ou des préparations pour nourrissons.

11. Dispositif d'allaitement selon la revendication 10, le canal à liquide comprenant en outre un capteur de débit (FS) configuré pour mesurer un débit de liquide dans le canal à liquide ou un moyen de pression (PM) configuré pour produire un écoulement de liquide sous pression depuis le récipient jusqu'à l'orifice du mamelon artificiel.

12. Dispositif d'allaitement selon la revendication 11, dans lequel le capteur de débit ou le moyen de pression sont configurés pour recevoir/transmettre des données à l'aide du moyen de transmission de données selon la revendication 2 et/ou du moyen de traitement selon la revendication 5.

13. Dispositif d'allaitement selon la revendication 2, dans lequel le moyen de transmission de données est un moyen de transmission de données sans fil et le dispositif d'allaitement comprend en outre un moyen de stockage d'énergie (EM) configuré pour alimenter un ou plusieurs parmi, mais sans s'y limiter, le capteur de pression, le système de résilience de mamelon et/ou le moyen de traitement selon la revendication 5.

14. Procédé d'identification de schémas de succion par un nourrisson, le procédé comprenant les étapes consistant à

    - fournir un dispositif d'allaitement (1) selon l'une quelconque des revendications 1 à 13,
    - positionner le mamelon artificiel (10) à l'intérieur de la bouche d'un nourrisson,
    - mesurer la succion sur le mamelon artificiel par le nourrisson,
    - déterminer un ou plusieurs schémas relatifs à la succion par le nourrisson, exerçant une pression sur le mamelon artificiel et la cavité (11, 14) pendant la succion.

15. Kit d'analyse d'allaitement pour un nourrisson, le kit comprenant

    - un dispositif d'allaitement selon l'une quelconque des revendications 1 à 13,
    - un produit programme informatique permettant à un système informatique d'identifier des schémas de succion sur la base de données fournies par le dispositif d'allaitement,

le produit programme informatique fournissant des données et/ou une aide à la décision à un professionnel de santé, en ce qui concerne le nourrissage de nourrisson.

Fig. 1

Fig. 2

11

1

F_C

BD

RS

PS

EM

DTM

Proc_M

Fig. 3

Fig. 4

Fig. 5

Fig. 6

Fig. 7

Fig. 8

Fig. 9

Fig. 10

**EP 4 436 476 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20150196247 A1
- WO 2010046812 A1